(19) Europäisches Patentamt
European Patent Office
Office européen des brevets

(11) Veröffentlichungsnummer: **0 017 998**
**B2**

(12) # NEUE EUROPÄISCHE PATENTSCHRIFT

(45) Veröffentlichungstag der neuen Patentschrift: **26.10.88**

(51) Int. Cl.⁴: **C 08 G 18/18,** C 07 C 119/042

(21) Anmeldenummer: **80102101.5**

(22) Anmeldetag: **18.04.80**

(54) **Verfahren zur kontinuierlichen Herstellung von Isocyanuratgruppen enhaltenden (cyclo)-aliphatischen Polyisocyanaten.**

(30) Priorität: **21.04.79 DE 2916201**

(43) Veröffentlichungstag der Anmeldung: **29.10.80 Patentblatt 80/22**

(45) Bekanntmachung des Hinweises auf die Patenterteilung: **11.04.84 Patentblatt 84/15**

(45) Bekanntmachung des Hinweises auf die Entscheidung über den Einspruch: **26.10.88 Patentblatt 88/43**

(84) Benannte Vertragsstaaten: **BE CH DE FR GB IT LI LU NL SE**

(73) Patentinhaber: **HÜLS AKTIENGESELLSCHAFT, Patentabteilung / PB 15 - Postfach 13 20, D-4370 Marl 1 (DE)**

(72) Erfinder: **Disteldorf, Josef, Dr., Am Sengenhoff 2a, D-4690 Herne 1 (DE)**
Erfinder: **Hübel, Werner, Dr., Am Birnenbruch 34, D-4690 Herne 1 (DE)**
Erfinder: **Wolf, Elmar, Dr., Am Böckenbusch 3a, D-4690 Herne 2 (DE)**

(74) Vertreter: **Steil, Hanna, Dipl.-Chem. et al, RSP PATENTE - PB 15 Postfach 1320, D-4370 Marl 1 (DE)**

(56) Entgegenhaltungen:
**EP - A - 0 003 765**
**EP - A - 0 015 559**
**DE - A - 1 150 080**
**DE - A - 2 525 017**
**DE - A - 2 527 242**
**DE - A - 2 631 733**
**DE - A - 2 641 380**
**DE - A - 2 644 684**
**DE - A - 2 806 731**
**DE - A - 3 219 608**
**GB - A - 1 386 399**
**US - A - 3 862 150**

(56) Entgegenhaltungen: (Fortsetzung)
**US - A - 3 980 594**
**US - A - 3 980 594**
**US - A - 4 040 992**

**G. u. H.V. ADOLPHI, Grundzüge der Verfahrenstechnik, 1970, Deutscher Verlag für Grundstoffindustrie, Leipzig, S. 259-260, 265-270**

EP 0 017 998 B2

**Beschreibung**

Die Trimerisierung von Isocyanaten ist eine bekannte Reaktion. Als Katalysatoren für die Trimerisierung von organischen Isocyanaten werden in der Literatur eine Vielzahl von chemisch sehr verschiedenartigen Verbindungen beschrieben. So können als Trimerisierungskatalysatoren Metallverbindungen aus der Gruppe Salze, Basen und homöopolare Metallverbindungen, wie Metallnaphthenate, Na-Benzoat in Dimethylformamid (DMF), Erdalkaliacetate, -formiate und -carbonate, Metallalkoxide, $AlCl_3$ und Fe-Acetylacetonat eingesetzt werden. Diese lassen sich aus den Reaktionsprodukten, den Isocyanuraten, nur sehr schwierig, bzw. schlecht oder überhaupt nicht abtrennen. Sie verbleiben vielmehr in den Reaktionsprodukten.

Aus der Gruppe der Basen und Salze haben vor allem quarternäre Ammoniumbasen und quarternäre Ammoniumsalze als Trimerisierungskatalysatoren für Isocyanate zunehmendes Interesse gefunden. So werden beispielsweise in der GB-PS 837 120 quarternäre Ammoniumbasen, in der US-PS 3 980 594 quarternäre Ammoniumsalze anorganischer und organischer O-Säuren mit einem pK von mindestens 2 (in wässriger Lösung), in der US-PS 3 862 150 quarternäre Ammoniumsalze aus tertiären Aminen und alpha-substituierten Carbonsäuren als Trimerisierungskatalysatoren für Isocyanate beschrieben. In der DE-OS 2 631 733 (US 4 040 992) wird ein Verfahren zur Förderung von Kondensations- und/oder Polymerisationsreaktionen von organischen Isocyanaten beschrieben, wobei man zur Förderung der Reaktion der organischen Isocyanate eine katalytische Menge eines quarternären N-(Hydroxyalkyl)-ammoniumsalzes der allgemeinen Formel

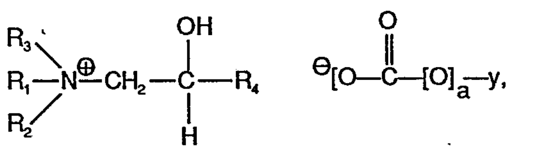

in der a 0 oder 1; $R_1$, $R_2$, $R_3$, $R_4$ z.B. gleiche oder verschiedene aliphatische, cycloaliphatische, araliphatische Kohlenwasserstoffreste und Y H oder Alkyl bedeuten, einsetzt.

Diese quarternären Ammoniumsalze dienen vor allem zur Herstellung von geschäumten Kunststoffen durch Umsetzen von aromatischen Diisocyanaten mit Polyolen. Im Gegensatz zu den meisten für die Herstellung von Isocyanuratschäumen vorgeschlagenen bekannten Katalysatoren fördern die in der DE-OS 2 631 733 beschriebenen keine zu schnelle Reaktion des Polyols mit dem Isocyanat auf Kosten der Trimerisierung des Isocyanats. Man erhält dadurch Polyisocyanurat-Polyurethanschäume mit gleichmässigem Aufbau (keine «Nester-Bildung»).

Bei den aromatischen Diisocyanaten und Polyolen mit Hilfe der quarternären Ammoniumsalze hergestellten geschäumten Kunststoffe handelt es sich um Produkte, die ausser geringe Mengen Rest-OH oder -NCO keine reaktiven Gruppen mehr enthalten. Es ist somit nicht erforderlich, den Katalysator nach der Reaktion aus dem Reaktionsprodukt zu entfernen.

Bei der Herstellung von Isocyanuraten durch Trimerisierung eines Isocyanats mit mehr als einer NCO-Gruppe im Molekül muss dagegen sehr darauf geachtet werden, dass der Trimerisierungskatalysator nach der Reaktion aus dem Reaktionsprodukt (Isocyanuratgruppe enthaltendes Polyisocyanat) entfernt bzw. desaktiviert wird. Beim Erhitzen reagieren nämlich die freien NCO-Gruppen dieser Isocyanurate in Gegenwart des Trimerisierungskatalysators noch weiter. Es bilden sich höhermolekulare, Isocyanuratringe enthaltende Polyisocyanate. Mit zunehmender Reaktion tritt sogar Vernetzung ein. Aus diesem Grunde ist es auch verständlich, warum ein noch katalysatorhaltiges Isocyanurat, das noch über Alkylengruppe am Triazinring gebundene NCO-Gruppen enthält, zur gezielten Weiterreaktion mit Polyolen oder Polyaminen nicht eingesetzt werden kann.

Das Ziel bei jeglicher Herstellung von Isocyanuraten aus Diisocyanaten muss daher die vollständige Entfernung des Katalysators nach beendeter Trimerisierung sein. Aus diesem Grunde scheiden die in der DE-OS 2 631 733 beanspruchten Katalysatoren für die Herstellung von Trimerisaten aus Diisocyanaten, die zur Herstellung eines kalthärtenden lösungsmittelhaltigen Polyurethan-2-Komponentenlacks Verwendung finden, aus. Versucht man nämlich, aliphatische wie auch aromatische Diisocyanate entsprechend den in der DE-OS 2 631 733 gemachten Angaben zu trimerisieren, so stellt man fest, dass bei Zugabe des Katalysators zum Diisocyanat sich ein Niederschlag bildet. Dieser verschwindet auch beim Erhitzen auf 40–60°C nicht; bei dieser Temperatur erfolgt unter starker Wärmeentwicklung die Isocyanuratbildung. Das Reaktionsgemisch erhitzt sich auch bei intensiver Kühlung dabei auf ca. 100°C. Der Katalysator ist auch im Reaktionsprodukt noch nachweisbar. Der Katalysator ist über eine OH/NCO-Reaktion im Reaktionsprodukt eingebaut und lässt sich aus diesem auch nicht abtrennen. Er beeinträchtigt einmal stark die Lagerstabilität der Isocyanuratgruppen enthaltenden Isocyanate und zum anderen deren gezielte kontrollierte Weiterreaktion mit Polyolen.

Weiterhin stellt man fest, dass die nach DE-OS 2 631 733 trimerisierten aliphatischen oder aromatischen Diisocyanate unangenehm nach Aminen riechen. Diisocyanate lassen sich zwar im Labormassstab nach den in der DE-OS 2 631 733 gemachten Angaben trimerisieren, wobei gemäss der Lehre der DE-OS 2 631 733 für die stark reaktiven aromatischen Diisocyanate Katalysatormengen von ca. 1–2 Gew.%, bezogen auf Diisocyanat, eingesetzt werden, was für die reaktionsträgeren (cyclo)-aliphatischen Diisocyanate in logischer Konsequenz das Erfordernis noch weit höherer Katalysatormengen erwarten lässt. Solche trimerisierten Diisocyanate scheiden allerdings für die Herstellung eines lösungsmittelhaltigen 2-Komponenten-PUR-Lackes schon aus den bereits erwähnten Gründen aus.

Eine wirtschaftliche Trimerisierung von Polyisocyanaten, speziell von Diisocyanaten, mittels der in der DE-OS 2 631 733 beschriebenen Katalysatoren in technischen Mengen scheitert:

1. an der grossen abzuführenden Reaktionswärme und
2. am Katalysatorniederschlag im Reaktionsprodukt und den sich daraus ergebenden Auswirkungen auf das trimerisierte Diisocyanat.

Des weiteren ist gemäss DE-PS 2 644 684 ein Verfahren zur diskontinuierlichen Herstellung von partiell trimerisierten (cyclo)-aliphatischen Diisocyanaten, z.B. Isophorondiisocyanat (IPDI) in Gegenwart von 0,1 bis 1 Gew.% eines Katalysatorsystems Olefinoxid/N.N′-endoäthylenpiperazin bekannt. Die Reaktion findet bei 80–200°C, insbesondere 120–180°C statt, die Reaktionszeit dauert 1–3 Stunden; bei einem Umsatz von 50% wird die Reaktion abgebrochen und nicht umgesetztes Monomer und Katalysator durch Dünnschichtdestillation entfernt. Das Verfahren hat den Nachteil, dass es bei der Abtrennung von Monomeren, z.B. IPDI, in regelmässigen Abständen von ca. 1–2 Wochen zu Ablagerungen und Verstopfungen in den Destillationsleitungen kommt und zudem im Öl der Vakuumpumpen eine Verharzung stattfindet.

So wurde weiterhin nach einem wirtschaftlichen Verfahren zur Trimerisierung von (cyclo)-aliphatischen Isocyanaten gesucht, das zudem zu einer problemlosen Isolierung des Reaktionsproduktes führt.

Gegenstand der Erfindung ist daher ein Verfahren zur kontinuierlichen Herstellung von Isocyanuratgruppen enthaltenden (cyclo)-aliphatischen Polyisocyanaten mit einem Gehalt an freien NCO-Gruppen zwischen 10 und 22% und einem Restmonomergehalt von weniger als 0,7% durch Trimerisierung von Diisocyanaten in Gegenwart von quarternären Ammoniumsalzen organischer Säuren der allgemeinen Formel

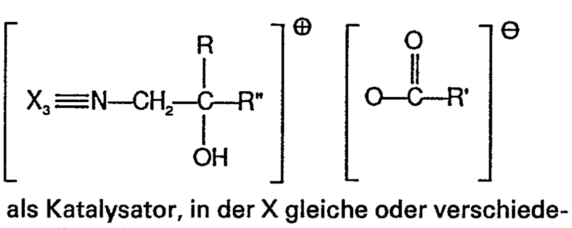

als Katalysator, in der X gleiche oder verschiedene aliphatische, cycloaliphatische, araliphatische oder heterocyclische Kohlenwasserstoffreste eines quarternären Ammonium-Stickstoffs sind oder 2X mit dem quarternären Stickstoff einen gegebenfalls ein oder mehr Heteroatome enthaltenden Ring bzw. 3X über ein gemeinsames Heteroatom mit dem quarternären Stickstoff einen Ring bilden, R ein Rest aus der Gruppe Alkyl-, Cycloalkyl- und Aralkyl- sowie R + R″ gemeinsam ein $C_4$-$C_7$-Alkylenrest, R′ Wasserstoff oder ein $C_1$-$C_{12}$-Alkylrest ist und R″ = R oder Wasserstoff ist, welches dadurch gekennzeichnet ist, dass man den Trimerisierungskatalysator in einer Menge von 0,02 bis 0,1 Gew.%, bezogen auf das Gewicht der zu trimerisierenden Verbindung einsetzt und die Trimerisierung in einer Reaktionsschlange in einem Temperaturbereich von 40–120°C, vorzugsweise 60–90°C, nur bis zu einem Umsatz von 45% durchführt, die Reaktion abbricht und das unumgesetzte Diisocyanat durch Dünnschichtverdampfung entfernt.

Der Trimerisierung lässt sich dann bei diesen Temperaturen innerhalb von 1–60 Minuten durchführen.

Dieser Effekt war überraschend und unerwartet, da man von vornherein bei einer solchen geringen Katalysatorkonzentration mit keiner Aktivität des Katalysators mehr rechnen konnte.

Durch die Trimerisierung gemäss dem vorliegenden Verfahren werden Verbindungen erhalten, die 1 oder mehr Isocyanuratringe haben. Verbindungen dieser Art sind in der Literatur beschrieben.

Die Trimerisierung wird kontinuierlich durchgeführt.

Für die Herstellung von monomerfreien Isocyanuraten kommen grundsätzlich zwei Verfahren in Frage:

1. vollständige Umsetzung des Diisocyanats in Substanz und Entfernen des Katalysators durch Extraktion mit einem Lösungsmittel, in dem das Isocyanurat kaum, das Diisocyanat dagegen gut löslich ist und
2. teilweise Umsetzung des Diisocyanats und Entfernung des nicht umgesetzten Diisocyanats im Vakuum durch Dünnschichtdestillaltion.

Das erfindungsgemässe Verfahren gehört zur unter 2. skizzierten Verfahrensweise. Für die Herstellung des diisocyanatfreien Isocyanurats aus dem Diisocyanat bedeutet das, dass man das Diisocyanat bis zu einem Isocyanuratgehalt, der die Förderung des Reaktionsgemisches im flüssigen Zustand noch zulässt, trimerisiert und in einem nachfolgenden Verfahrensschritt das freie Diisocyanat im Vakuum durch Dünnschichtdestillation abtrennt.

Die Isocyanurate gemäss der Erfindung haben einen NCO-Gehalt von ca. 10–22% und einen Monomergehalt < 0,7%.

Erfindungsgemäss wird die Trimerisierung von Diisocyanaten in einer kontinuierlich arbeitenden Reaktionsschlange unter kontinuierlicher, gleichzeitiger Zudosierung des Diisocyanats und des Trimerisierungskatalysators bei 40–120°C und innerhalb von 1–7 Minuten durchgeführt. Eine Reaktionsschlange mit einem kleinen Durchmesser führt zur Erreichung hoher Strömungsgeschwindigkeiten. Weiterhin ist es sehr vorteilhaft, das Diisocyanat/Katalysatorgemisch vor Eintritt in die Reaktionsschlange auf ca. 60°C zu erhitzen.

Die Reaktionsschlange kann beispielsweise auch in 2–3 Zonen geteilt sein, die unabhängig voneinander geheizt oder gegebenenfalls gekühlt werden können, wobei in der 1. Zone das Vorwärmen der Einsatzstoffe (Diisocyanat + Katalysator) auf Reaktionstemperatur, in der zweiten Aufrechterhaltung der Reaktionstemperatur durch teilweises Abführen der Reaktionswärme und in

der 3. Zone die Abkühlung des Reaktionsgemisches erfolgt. Sollte das Reaktionsgemisch ohne Zwischenlagerung gleich der Dünnschichtdestillation zugeführt werden, kann die Abkühlung in der 3. Zone entfallen.

Entscheidend bei dieser kontinuierlichen Herstellung von trimerisierten Diisocyanaten ist die Zudosierung des Katalysators. Als besonders zweckmässig hat sich erwiesen, wenn vor Eintritt in die Reaktionsschlange die Einsatzprodukte intensiv gemischt werden.

Die Temperatur der Reaktionsschlangenabschnitte wählt man zweckmässigerweise so, dass die Vorwärmzone ca. 40–60°C, die Reaktionszone 70–120°C, vorzugsweise 70–90°C, und die Abkühlzone 20–40°C hat. Bei einem Durchsatz von 40–120 kg/h Diisocyanat pro 0,5 $cm^2$ Reaktorquerschnitt lässt sich dann leicht ein Diisocyanat-Umsatz von ca. 35–45% erzielen. Diese Temperaturbedingungen sind jedoch jeweils den erforderlichen Bedingungen für das zu trimerisierende Diisocyanat anzupassen.

Die Verweilzeit des Diisocyanat-Katalysator-Gemisches in der Reaktionsschlange beträgt ca. 1–7 Minuten. Innerhalb dieser Zeit sind von dem Diisocyanat ca. 35–45% in die trimerisierte Form überführt worden. Zur Entfernung des nicht umgesetzten Diisocyanats wird das Reaktionsgemisch, wie bereits erwähnt, dann einer Dünnschichtverdampfung unterworfen. Für das erfindungsgemässe Verfahren geeignete Katalysatoren sind alle quarternären Ammoniumsalze, wie sie in der vorveröffentlichten DE-OS 2 631 733 beschrieben werden. Zur exakteren Dosierung der geringen Katalysatormengen kann es vorteilhaft sein, den Katalysator in einem geeigneten organischen Lösungsmittel zu lösen. Geeignet sind solche, die keine funktionellen Gruppen tragen, welche Reaktionen mit den Isocyanatgruppen bei den Trimerisierungsbedingungen eingehen können.

Das erfindungsgemässe Verfahren wird vorzugsweise weitgehend lösungsmittelfrei durchgeführt. Die obgenannten geringen, gegebenenfalls zur Auflösung des Katalysators dienenden Lösungsmittelmengen stören die Trimerisierung nicht.

Als Ausgangsverbindung, die zur Trimerisierung nach dem erfindungsgemässen Verfahren eingesetzt werden können, eignen sich aliphatische und cycloaliphatische Diisocyanate, wie sie beispielsweise in Houben-Weyl, Methoden der organischen Chemie, Band 14/2, Seite 61–70 und dem Artikel von W. Siefken in Justus Liebigs Annalen der Chemie 562, 75–136, beschrieben werden, wie 1,2-Äthylendiisocyanat, 1,4-Tetramethylendiisocyanat, 1,6-Hexamethylendiisocyanat, 2,2,4- bzw. 2,4,4-Trimethyl-1-1,6-hexamethylendiisocyanat (TMDI), 1,9-Diisocyanato-5-methyl-nonan, 1,8-Diisocyanato-2,4-dimethyloctan, 1,12-Dodecandiisocyanat, ω,ω'-Diisocyanatodipropyläther, Cyclobuten-1,3-diisocyanat, Cyclohexan-1,3- und 1,4-diisocyanat, 3-Isocyanatomethyl-3,5,5-trimethyl-cyclohexylisocyanat (Isophorondiisocyanat, IPDI), 1,4-Diisocyanatomethyl-2,3,5,6-tetramethylcyclohexan, Decahydro-8-methyl-(1,4-methanol-naphthalin-2 (oder 3) 5-ylendimethylendiisocyanat, Hexahydro-4,7-methano-indan-1 (oder 2) 5 (oder 6) ylendimethylendiisocyanat, Hexahydro-4,7-methanoindan-1 (oder 2) 5 (oder 6) ylendiisocyanat, 2,4- und 2,6-Hexahydrotoluylendiisocyanat, Perhydro-2,4' und/oder -4,4' -diphenyl-methandiisocyanat, 4,4' -Diisocyanato-3,3', 5, 5' -tetramethyl-dicyclohexylmethan, 4,4' -Diisocyanato-2,2', 3,3', 5,5', 6,6' -octomethyl-dicyclohexylmethan, ω,ω' -Diisocyanato-1,4-diäthylbenzol, 1,4-Diisocyanatomethyl-2,3,5,6-tetramethyl-benzol, m-Xylylen-diisocyanat sowie beliebige Gemische dieser Verbindungen. Weitere geeignete Isocyanate werden in dem genannten Artikel in den Annalen auf Seite 122 f beschrieben. Besonders bevorzugt werden in der Regel die technisch leicht zugängigen aliphatischen und cycloaliphatischen Diisocyanate sowie deren isomere Gemische.

Die erfindungsgemäss hergestellten isocyanuratgruppenhaltigen Isocyanate stellen wertvolle Zwischenprodukte zur Herstellung von Polyurethanen dar.

A. Herstellung der als Katalysatoren eingesetzten N-(Hydroxyalkyl)-ammoniumsalze

Beispiel A1

In einen mit Rührer, Rückflusskühler und Tropftrichter versehenen 1 l-Dreihalskolben wurden 232 g Dipropylenglykol (DPG) und 90 g Eisessig gegeben. In diese Mischung wurden dann bis zu einer Gewichtszunahme von 87 g Trimethylamin eingeleitet. Anschliessend wurden langsam bei 25°C unter intensiver Rührung 87 g Propylenoxid (PO) zugegeben. Nach Beendigung der PO-Zugabe wurde die Mischung bei Raumtemperatur über Nacht gerührt, dann im Vakuum innerhalb von 6 h bei 45°C die nicht umgesetzten flüchtigen Materialien abgezogen. Es blieb ein Rückstand mit einem Gewicht von 484 g zurück.

Unter Anwendung der oben beschriebenen Verfahrensweise wurden weitere Verbindungen hergestellt, deren Zusammensetzung in der folgenden Tabelle (Beispiel A2–A8) zusammengestellt ist.

Tabelle I
Trimerisierungskatalysatoren

| Beispiel | Amin | Säure | Alkylenoxid | Lösungsmittel |
|---|---|---|---|---|
| A2 | Trimethylamin | Essigsäure | Propylenoxid | Dipropylenglykol |
| A3 | Trimethylamin | Ameisensäure | Propylenoxid | Dipropylenglykol |
| A4 | Chinuclidin | Essigsäure | Glycidol | Dipropylenglykol |
| A5 | N,N'-Endo äthylen piperazin | Ameisensäure | Propylenoxid | keine |
| A8 | Trimethylamin | 2-Äthylhexan-säure | Propylenoxid | Dipropylenglykol |

Tabelle I (Fortsetzung)

| NMR-Analyse | | | |
|---|---|---|---|
| Mol-% quarternärer Stickstoff | Mol-% tertiärer Stickstoff | Mol-% Cipropylenglykol | Beispiel |
| 46,5 | 0,00 | 53,5 | A2 |
| 41,5 | 0,00 | 58,5 | A3 |
| 38,1 | 0,00 | 61,9 | A4 |
| 55,0-29,0 mono | 0,00 | 16,0 | A5 |
| 59,0 | 0,00 | 41,0 | A8 |

B. Trimerisierung von Diisocyanaten mit den unter A. beschriebenen Katalysatoren

Kontinuierliche Herstellung

Die kontinuierliche Herstellung von partiell trimerisierten Diisocyanaten (bis zu ca. 35-45%igen Umsätzen) erfolgte in einem Rohrschlangenreaktor bei 60-120°C und mit einer Verweilzeit von ca. 1-7 Minuten. Der Schlangenreaktor bestand aus zwei Heizzonen und einer Kühlzone, wobei in der 1. Heizzone zunächst das Vorwärmen der Einsatzstoffe (Diisocyanate + Katalysatoren) auf Reaktionstemperatur erfolgte und in der zweiten Zone die Reaktionstemperatur durch teilweises Abführen der Reaktionswärme auf dieser Reaktionstemperatur gehalten wurde; in der Kühlzone wurde das Reaktionsgemisch auf Temperaturen unterhalb von 40°C gekühlt.

Die in einem Vormischer intensiv gerührten Diisocyanat/Katalysator-Gemische traten mit ca. 30°C in die Aufheizschlange, die mit 85-90°C heissem Öl beheizt wurde. Nach dem Durchströmen der Aufheizschlange hatten die Diisocyanat-Katalysator-Gemische bei einer Verweilzeit von ca. 0,8-1,5 Minuten eine Temperatur von ca. 80-85°C und waren bereits zu 7,5-10% trimerisiert.

Die weitere Umsetzung der Diisocyanate von 7,5-10% auf maximal 35-45% erfolgte dann bei 80-90°C. Hierbei sind ca. 84 Kilojoule pro kg Durchsatz abzuführen. In der nachgeschalteten Kühlschlange wurde anschliessend das Reaktionsgemisch auf eine Temperatur unterhalb 40°C abgekühlt.

Die für diese Versuche zur Herstellung von partiell trimerisierten Diisocyanaten verwendete Schlangenkombination hatte folgende Dimensionierung:

| | Innendurchmesser (mm) | Länge (m) | Inhalt (l) |
|---|---|---|---|
| Aufheizschlange | 10 | 7 | 0,6 |
| | 6 | 3 | |
| Reaktionsschlange | 14 | 9 | 1,4 |
| Kühlschlange | 14 | 18 | 2,8 |

Nach Verlassen der Kühlschlange hatte das Reaktionsgemisch bei einer in den nachfolgenden Tabellen IIa + b angegebenen Verweilzeit einen NCO-Gehalt, der einem Diisocyanat-Umsatz von ca. 35-45% entsprach.

Der Umsatz zur trimerisierten Form kann durch laufende Kontrolle der Brechungsindices vorgenommen werden. Brechungsindices für IPDI im Bereich von etwa 1,4950-1,4990 entsprechen etwa einem Umsatz in der Grössenordnung von 35-45%.

Die Abtrennung der nicht umgesetzten Diisocyanate von der trimerisierten Form erfolgte kontinuierlich im Vakuum in einer Vor- und einer Hauptverdampferstufe, wobei die Destillate jeweils erneut zur Trimerisierung eingesetzt wurden.

Tabelle IIa
Kontinuierliche Trimerisierung verschiedener Diisocyanate

| Beispiel | eingesetztes Isocyanat | Katalysator (Gew.-%), bezogen auf eingesetztes Diisocyanat | | Durchsatz kg/h | Verweilzeit (min) in der Reaktionsschlange | Temperatur (°C) der Heizzone – HZ | | | Brechungsindex $n_D25$ nach | | | Dünnschichtdestillative Aufarbeitung des partiell trimerisierten Diisocyanats – Zs.-Setzung des Rückstands | |
|---|---|---|---|---|---|---|---|---|---|---|---|---|---|
| | | Gew.-% | Kat.-Beispiel | | | HZ 1 | HZ 2 | HZ 3 | HZ 1 | HZ 2 | HZ 3 | % NCO | Monomer-Gehalt |
| B1 | TMDI | 0,05 | A1 | 40 | 2,10 | 86 | 85 | 33 | 1,4690 | 1,4770 | 1,4775 | 16,9 | <0,6 |
| B2 | TMDI | 0,03 | A2 | 60 | 1,39 | 87 | 86 | 32 | 1,4683 | 1,4765 | 1,4770 | 16,8 | <0,6 |
| B3 | TMDI | 0,02 | A8 | 50 | 1,76 | 87 | 86 | 32 | 1,4672 | 1,4661 | 1,4755 | 17,0 | <0,7 |
| B4 | $OCN-(CH_2)_6-NCO$ | 0,04 | A1 | 70 | 1,20 | 86 | 86 | 33 | 1,4570 | 1,4661 | 1,4668 | 21,0 | <0,5 |
| B5 | $OCN-(CH_2)_6-NCO$ | 0,02 | A2 | 45 | 1,86 | 87 | 86 | 32 | 1,4564 | 1,4653 | 1,4658 | 21,4 | <0,5 |
| B6 | $OCN-(CH_2)_6-NCO$ | 0,05 | A3 | 49 | 1,77 | 88 | 87 | 33 | 1,4573 | 1,4668 | 1,4676 | 20,9 | <0,6 |
| B7 | | 0,06 | A2 | 45 | 1,86 | 87 | 86 | 32 | 1,5187 | 1,5261 | 1,5270 | 11,3 | <0,5 |
| B8 | 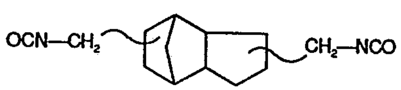 | 0,07 | A3 | 60 | 1,39 | 87 | 86 | 33 | 1,5193 | 1,5266 | 1,5273 | 11,5 | <0,6 |
| B9 | | 0,08 | A8 | 75 | 1,12 | 87 | 86 | 33 | 1,5198 | 1,5273 | 1,5283 | 11,5 | <0,6 |
| B10 | 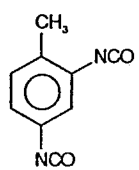 20 Gew.% 2,6- 80 Gew.% 2,4- | 0,01 | A1 | 60 | 1,39 | 87 | 86 | 33 | 1,5712 | 1,5781 | 1,5789 | 21,5 | <0,7 |
| | | 0,02 | A2 | 65 | 1,29 | 87 | 86 | 32 | 1,5729 | 1,5794 | 1,5803 | 21,0 | <0,6 |
| | | 0,01 | A8 | 60 | 1,39 | 86 | 85 | 32 | 1,5701 | 1,5778 | 1,5781 | 21,3 | <0,7 |
| B11 | 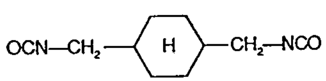 | 0,07 | A3 | 60 | 1,39 | 86 | 86 | 32 | 1,4901 | 1,4961 | 1,4970 | 19,5 | <0,7 |
| B12 | 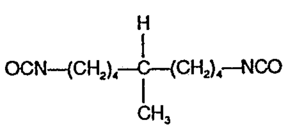 | 0,05 | A1 | 70 | 1,20 | 87 | 86 | 33 | 1,4610 | 1,4695 | 1,4706 | 15,4 | <0,6 |
| B13 | 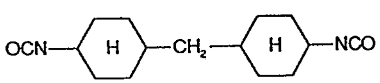 | 0,08 | A2 | 65 | 1,29 | 88 | 87 | 33 | 1,5025 | 1,5092 | 1,5097 | 14,3 | <0,6 |

Tabelle IIa (Fortsetzung)

| Temperatur (°C) der Heizzone – HZ | | | Brechungsindex $n_D^{25}$ nach | | | Dünnschichtdestillative Aufarbeitung des partiell trimerisierten Diisocyanats – Zs.-Setzung des Rückstands | |
|---|---|---|---|---|---|---|---|
| HZ 1 | HZ 2 | HZ 3 | HZ 1 | HZ 2 | HZ 3 | % NCO | Monomer-Gehalt |
| 86 | 85 | 33 | 1,4690 | 1,4770 | 1,4775 | 16,9 | < 0,6 |
| 87 | 86 | 32 | 1,4683 | 1,4765 | 1,4770 | 16,8 | < 0,6 |
| 87 | 86 | 32 | 1,4672 | 1,4661 | 1,4755 | 17,0 | < 0,7 |
| 86 | 86 | 33 | 1,4570 | 1,4661 | 1,4668 | 21,0 | < 0,5 |
| 87 | 86 | 32 | 1,4564 | 1,4653 | 1,4658 | 21,4 | < 0,5 |
| 88 | 87 | 33 | 1,4573 | 1,4668 | 1,4676 | 20,9 | < 0,6 |
| 87 | 86 | 32 | 1,5187 | 1,5261 | 1,5270 | 11,3 | < 0,5 |
| 87 | 86 | 33 | 1,5193 | 1,5266 | 1,5273 | 11,5 | < 0,6 |
| 87 | 86 | 33 | 1,5198 | 1,5273 | 1,5283 | 11,5 | < 0,6 |
| 87 | 86 | 33 | 1,5712 | 1,5781 | 1,5789 | 21,5 | < 0,7 |
| 87 | 86 | 32 | 1,5729 | 1,5794 | 1,5803 | 21,0 | < 0,6 |
| 86 | 85 | 32 | 1,5701 | 1,5778 | 1,5781 | 21,3 | < 0,7 |
| 86 | 86 | 32 | 1,4901 | 1,4961 | 1,4970 | 19,5 | < 0,7 |
| 87 | 86 | 33 | 1,4610 | 1,4695 | 1,4706 | 15,4 | < 0,6 |
| 88 | 87 | 33 | 1,5025 | 1,5092 | 1,5097 | 14,3 | < 0,6 |

Tabelle II b

Kontinuierliche Trimerisierung von IPDI in Abhängigkeit von Katalysator und Verweilzeit

| Beispiel | Katalysator bezogen auf IPDI | | Durchsatz kg/h | Verweilzeit (min) in Reakt.-Schlange | Temperatur (°C) Heizzone – HZ | | | Brechungsindex $n_D^{25}$ nach | | | Dünnschichtdestillative Aufarbeitung des partiell trimerisierten IPDI Zs. des Rückstandes | |
|---|---|---|---|---|---|---|---|---|---|---|---|---|
| | Gew. % | Kat.-Bsp. | | | HZ 1 | HZ 2 | HZ 3 | HZ 1 | Hz 2 | HZ 3 | % NCO | Monomer-Gehalt % |
| 14 | 0,080 | A1 | 45 | 1,86 | 85 | 85 | 34 | 1,4900 | 1,4961 | 1,4970 | 17,38 | 0,5 |
| 15 | 0,079 | A2 | 40 | 2,10 | 94 | 84 | 38 | 1,4905 | 1,4948 | 1,4952 | 17,50 | 0,5 |
| 16 | 0,062 | A2 | 60 | 1,39 | 85 | 85 | 34 | 1,4890 | 1,4958 | 1,4964 | 17,45 | 0,2 |
| 17 | 0,070 | A2 | 60 | 1,39 | 86 | 87 | 35 | 1,4888 | 1,4974 | 1,4981 | 17,25 | 0,3 |
| 18 | 0,081 | A2 | 75 | 1,12 | 86 | 87 | 39 | 1,4859 | 1,4934 | 1,4939 | 17,51 | 0,2 |
| 19 | 0,080 | A2 | 70 | 1,20 | 86 | 86 | 38 | 1,4870 | 1,4952 | 1,4965 | 17,3 | 0,3 |
| 20 | 0,055 | A2 | 40 | 2,10 | 80 | 80 | 32 | 1,4878 | 1,4959 | 1,4962 | 17,44 | 0,4 |
| 21 | 0,065 | A3 | 70 | 1,20 | 86 | 86 | 34 | 1,4891 | 1,4869 | 1,4975 | 17,43 | 0,4 |
| 22 | 0,057 | A8 | 40 | 2,10 | 85 | 85 | 33 | 1,4880 | 1,4956 | 1,4960 | 17,40 | 0,3 |
| 23 | 0,057 | A8 | 60 | 1,39 | 86 | 86 | 33 | 1,4870 | 1,4952 | 1,4958 | 17,42 | 0,4 |
| 24 | 0,062 | A8 | 65 | 1,29 | 87 | 86 | 34 | 1,4882 | 1,4960 | 1,4963 | 17,40 | 0,5 |

Die Herstellung der Trimerisierungskatalysatoren ist bekannt.

Bei den üblichen Verfahren zur Herstellung von N-(Hydroxy-alkyl)-ammonniumsalzen vermischt man äquivalente Mengen eines tertiären Amins, einer Carbonsäure und eines Alkylenoxids, was man vorzugsweise in einem geeigneten Lösungsmittel, wie Dipropylenglykol, durchführen kann. Die Reaktion wird dann bei einer Temperatur im Bereich von 25–60°C und bei etwa Atmosphärendruck durchgeführt, wobei man auch bei höheren Drücken arbeiten kann, beispielsweise bei Drükken von bis zu 35 Atmosphären. Das vorstehende Beispiel A1 verdeutlicht eine typische Herstellungsweise. Bei den weiteren Beispielen A2–A8 ist die gleiche Verfahrensweise angewandt worden, wobei auch geringfügige Änderungen hinsichtlich der Temperatur, der Auswahl des Lösungsmittels oder der Anwendung des Lösungsmittels möglich sind.

Als tertiäre Amine, die durch Umsetzen mit dem Alkylenoxid und der Carbonsäure zur Herstellung der erfindungsgemäss verwendeten Katalysatoren eingesetzt werden können, kann man jene Amine nennen, die eine bis drei Hydroxyalkylgruppen und ein oder mehrere Alkylgruppen, Cycloalkylgruppen oder Arylgruppen an das Stickstoffatom gebunden aufweisen. Besonders bevorzugte tertiäre Amine dieser Art sind unter anderem Trimethylamin, N-Dimethyl-N-hydroxyäthylamin, N-Benzyl-N-dimethylamin, Triäthylamin, N-Di-(hydroxyäthyl)-N-phenylamin, Triäthanolamin, N-Cyclohexyl-N-dimethylamin, N-Methylmorpholin, N-N′-Endoäthylenpiperazin-(1,4-Diaza-bicyclooctan-[2,2,2]) und Chinuclidin.

Von den Alkylenoxiden, die mit den tertiären Aminen umgesetzt werden können, sind besonders die aliphatischen hervorzuheben, wie Äthylenoxid, Propylenoxid, 1,2-Butylenoxid, Butadiendioxid, Methylglycidäther, Äthylglycidäther, Propylglycidäther, Alkylgliycidäther, n-Butylglycidäther, 2-Äthylhexylglycidäther, Diglycidäther u.a., Glycidol und Alkylenoxide mit längeren Ketten (die im Handel unter der Bezeichnung «Nedox» von Ashland Chemical Company erhältlich sind) der allgemeinen Formel

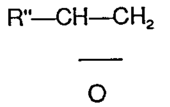

in der $R^a$ für eine langkettige Alkylgruppe oder eine Mischung solcher Gruppen mit bis zu 15 Kohlenstoffatomen steht, die cycloaliphatischen, Cyclohexenoxid, Cyclopentadienoxid, Vinylcyclohexendioxid, Dicyclopentadiendioxid sowie die aromatischen Epoxide, wie Styroloxid, Phenylglycidäther u.a.

Das Anion des gewünschten quarternären Ammoniumsalzes kann irgendeine Carbonsäure sein.

So erhält man die Verbindung der genannten allgemeinen Formel mit Fettsäuren mit kurzer bis langer Kette, mit substituierten aliphatischen Säuren. Besonders bevorzugte Säuren sind Ameisensäure, Essigsäure, Hexansäure sowie die geradkettigen oder verzweigten Heptansäuren, Octansäuren, 2-Äthylenhexansäure, Decansäuren und Hexadecansäuren; Neosäure, wie 3,3-Dimethyl-butansäure u.a.

**Patentansprüche**

1. Verfahren zur kontinuierlichen Herstellung von Isocyanuratgruppen enthaltenden (cyclo)-aliphatischen Polyisocyanaten mit einem Gehalt an freien NCO-Gruppen zwischen 10 und 22% und einem Restmonomergehalt von weniger als 0,7% durch Trimerisierung von Diisocyanaten in Gegenwart von quarternären Ammoniumsalzen organischer Säuren der allgemeinen Formel

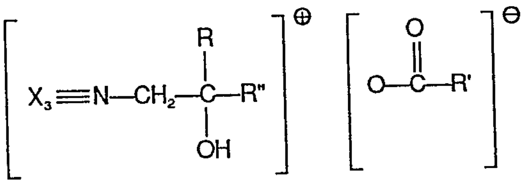

als Katalysator, in der X gleiche oder verschiedene aliphatische, cycloaliphatische, araliphatische oder heterocyclische Kohlenwasserstoffreste eines quarternären Ammonium-Stickstoffs sind oder 2X mit dem quarternären Stickstoff einen gegebenenfalls ein oder mehr Heteroatome enthaltenden Ring bzw. 3X über ein gemeinsames Heteroatom mit dem quarternären Stickstoff einen Ring bilden, R ein Rest aus der Gruppe Alkyl-, Cycloalkyl- und Aralkyl- sowie R + R″ gemeinsam ein $C_4$-$C_7$-Alkylenrest, R′ Wasserstoff oder ein $C_1$-$C_{12}$-Alkylrest ist und R″ = R oder Wasserstoff ist, dadurch gekennzeichnet, dass man den Trimerisierungskatalysator in einer Menge von 0,02 bis 0,1 Gew.%, bezogen auf das Gewicht der zu trimerisierenden Verbindung einsetzt und die Trimerisierung in einer Reaktionsschlange in einem Temperaturbereich von 40–120°C, vorzugsweise 60–90°C, nur bis zu einem Umsatz von 45% durchführt, die Reaktion abbricht und das unumgesetzte Diisocyanat durch Dünnschichtverdampfung entfernt.

2. Verfahren nach Anspruch 1, dadurch gekennzeichnet, dass man zur Trimerisierung aliphatische Diisocyanate einsetzt.

3. Verfahren nach Anspruch 1, dadurch gekennzeichnet, dass man zur Trimerisierung cycloaliphatische Diisocyanate einsetzt.

4. Verfahren nach Anspruch 3, dadurch gekennzeichnet, dass das cycloaliphatische Diisocyanat 3-Isocyanatomethyl-3,5,5-trimethyl-cyclohexylisocyanat ist.

**Claims**

1. Process for continuous preparation of (cyclo)-aliphatic polyisocyanates containing isocyanurate groups and having a content of free NCO groups of between 10 and 22% and a residual momomer content of less than 0.7%, by trimerisation of diisocyanates in the presence of quaternary ammonium salts of organic acids of the general formula

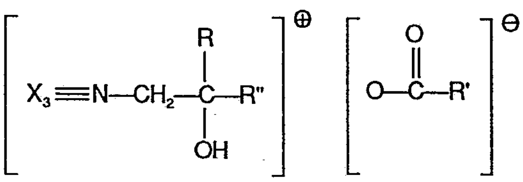

as a catalyst, in which X are identical or different aliphatic, cycloaliphatic, araliphatic or heterocyclic hydrocarbon radicals of a quaternary ammonium nitrogen, or 2X, together with the quaternary nitrogen, form a ring which optionally contains one or more hetero atoms or 3X, together with the quaternary nitrogen, form a ring via a common hetero atom, R is a radical from the group comprising alkyl, cycloalkyl and aralkyl radicals, and R + R″ together form a $C_4$-$C_7$-alkylene radical, R′ is hydrogen or a $C_1$-$C_{12}$-alkyl radical and R″ is R or hydrogen, characterised in that the trimerisation catalyst is employed in an amount of 0.02 to 0.1% by weight, based on the weight of the compound to be trimerised, and the trimerisation is carried out in a reaction coil in a temperature range of 40–120°C, preferably 60–90°C, only up to a conversion of 45%, the reaction is terminated and the unreacted diisocyanate is removed by thin-film evaporation.

2. Process according to Claim 1, characterised in that aliphatic diisocyanates are employed for the trimerisation.

3. Process according to Claim 1, characterised in that cycloaliphatic diisocyanates are employed for the trimerisation.

4. Process according to Claim 3, characterised in that the cycloaliphatic diisocyanate is 3-isocyanatomethyl-3,5,5-trimethylcyclohexyl isocyanate.

**Revendications**

1. Procédé pour l'obtention en continu de polyisocyanates (cyclo) aliphatiques contenant des groupes isocyanuriques ayant une teneur en groupes N = C = O libres située entre 10 et 22% et une teneur résiduelle en Monomère de moins de 0,7%, par trimérisation de Diisocyanates en présence de sels d'Ammonium quaternaire d'acides organiques, de formule générale:

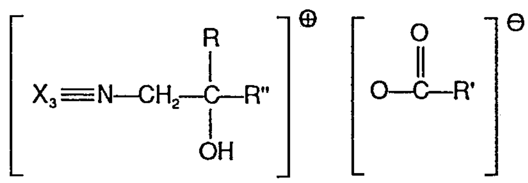

comme catalyseur, dans laquelle X sont des restes hydrocarbonés aliphatiques, cycloaliphatiques, arylaliphatiques ou hétérocycliques, semblables ou différents, à l'azote d'un ammonium quaternaire ou 2X forment un cycle avec l'azote quaternaire, contenant éventuellement un ou plusieurs hétéroatomes ou 3X forment un cycle par l'intermédiaire d'un hétéroatome commun, avec l'azote quaternaire, R est un reste choisi dans le groupe formé d'alcoyle, cycloalcoyle et aralcoyle ainsi que R et R″ ensemble forment un reste alcoylène en $C_4$ à $C_7$, R′ est de l'hydrogène ou un reste alcoyle en $C_1$ à $C_{12}$et R″ est égal à R ou est de l'hydrogène, caractérisé en ce que l'on met en œuvre le catalyseur de Trimérisation en une quantité de 0,02 à 0,1% en poids rapportée au poids du composé à trimériser et la trimérisation est effectuée dans un serpentin de réaction, dans une zone de températures de 40 à 120°C, de préférence à 60–90°C, seulement jusqu'à un taux de conversion de 45%, interrompt la réaction et élimine le Diisocyanate qui n'a pas été transformé par évaporation en couche mince.

2. Procédé selon la revendication 1, caractérisé en ce que l'on met en œuvre des diisocyanates aliphatiques pour la Trimérisation.

3. Procédé selon la revendication 1, caractérisé en ce que l'on met en œuvre des diisocyanates cycloaliphatiques pour la Trimérisation.

4. Procédé selon la revendication 3, caractérisé en ce que la diisocyanate cycloaliphatique est l'isocyanate de 3-Isocyanatométhyl-3,5,5-triméthylcyclohexyle.